# EUROPEAN PATENT SPECIFICATION

(11) **EP 0 287 977 B1**
(45) Date of publication and mention of the grant of the patent: **29.06.1994**
(21) Application number: 88106095.8
(22) Date of filing: 16.04.1988
(51) Int. Cl.: C07D 495/04, C07D 491/04, C07D 333/36, C07D 309/28, C07D 335/02, A61K 31/47

(54) **Fused heteroalkylene quinolinamines, a process and intermediates for their preparation and their use as medicaments**
Kondensierte Heteroalkylenchinolinamine, ein Verfahren und Zwischenprodukte zu ihrer Herstellung und ihre Verwendung als Arzneimittel
Hétéroalkylène quinoléine amines, un procédé et intermédiaires pour leur préparation et leur utilisation comme médicaments

(30) Priority: 23.04.1987 US 41562
(43) Date of publication of application: 26.10.1988
(73) Proprietor: HOECHST-ROUSSEL PHARMACEUTICALS INCORPORATED, Somerville New Jersey 08876 (US)
(72) Inventor: Shutske, Gregory Michael, Somerset, N.J.08873 (US); Effland, Richard Charles, Bridgewater, N.J. 08807 (US)
(74) Representative: Losert, Wolfgang Dr.

(56) References cited:
- US-A- 3 674 790
- US-A- 3 987 047
- CHEMICAL ABSTRACTS, vol. 84, no. 19, May 10, 1976, Columbus, Ohio, USA KHALDEEVA, V. A. ; KONSHIN, M. E. "Synthesis of substituted 9- (arylamino)-2-methyl-1,2,3,4-tetrahydro-2 - azaacridines" page 487, column 2, abstract-no. 135517d & Izv. Vyssh. Uchebn. Zaved., Khim. Khim. Tekhnol. 1976, 19 (1), 163

## Description

This invention relates to compounds having the formula 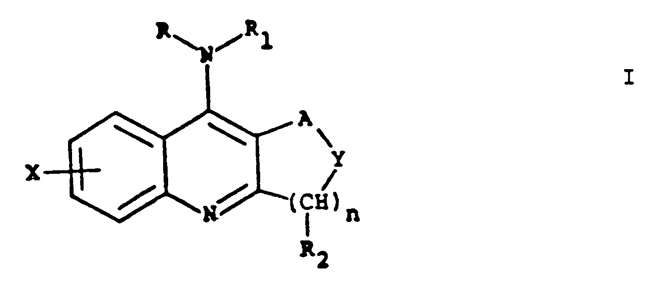
wherein n is 1-4; R is hydrogen, C₆-C₆-alkyl or C₁-C₆-alkylcarbonyl; R₁ is hydrogen, C₁-C₆-alkyl, C₁-C₆-alkylcarbonyl, di(C₁-C₆-alkyl)amino-C₁-C₆-alkyl or phenyl, phenyl-C₁-C₆-alkyl, diphenyl-C₁-C₆-alkyl oxygen-bridged phenyl-C₁-C₆-alkyl, or oxygen-bridged diphenyl-C₁-C₆-alkyl where the phenyl may be substituted with 1, 2 or 3 substituents each of which being independently C₁-C₆-alkyl, C₁-C₆-alkoxy, halogen, hydroxy, trifluoromethyl; phenoxy or benzyloxy; A is a direct bond or (CHR₃)ₘ, m being 1-3; X is hydrogen, C₁-C₆-alkyl, C₃-C₇-cycloalkyl, C₁-C₆-alkoxy, halogen, hydroxy, nitro, trifluoromethyl, formyl, C₁-C₆-alkylcarbonyl, phenylcarbonyl wherein the phenyl my be substituted as indicated above, -SH, C₁-C₆-alkylthio, -NHCOR₄ or -NR₅R₆, R₄ being hydrogen or C₁-C₆-alkyl, and R₅ and R₆ being independently hydrogen, C₁-C₆-alkyl or C₃-C₇-cycloalkyl; Y is O, S or NR₇; and each R₂, each R₃ and R₇ are independently hydrogen or C₁-C₆-alkyl, or taken two at a time form a methylene or ethylene group constituting a part of a ring of at least five atoms; with the proviso that when A is CH₂, Y is NCH₃, (CHR₂)ₙ, is CH₂CH₂, X is H, CH₃, Cl, Br or NO₂ and R is H, R₁ is not H, methyl, ethyl, propyl, butyl or benzyl, that when A is -CH₂- or -CHR'-, Y is NH or NR' and (CHR₂)ₙ is -CH₂CH₂- or -CH₂CHR'-, the group -NRR₁ is not -NH₂, -NHC₆H₅ or di-C₁-C₆-alkylamino-C₁-C₆-alkylamino, each R' being independently C₁-C₆-alkyl, that when A is CH₂, Y is NH or NR' and (CHR₂)ₙ is -(CH₂)₃- or -CHR'CH₂CH₂-, the group -NRR₁ is not -NH₂ and that when A is -CH₂CH₂-, Y is NH or NR' and (CHR₂)ₙ is -CH₂CH₂- or -CHR'CH₂-, the group -NRR₁ is not -NH₂; stereo, optical and geometrical isomers thereof, which are useful for enhancing memory, methods for synthesizing them,and pharmaceutical compositions comprising an effective memory enhancing amount of such a compound.

This invention also relates to compounds having the formulas 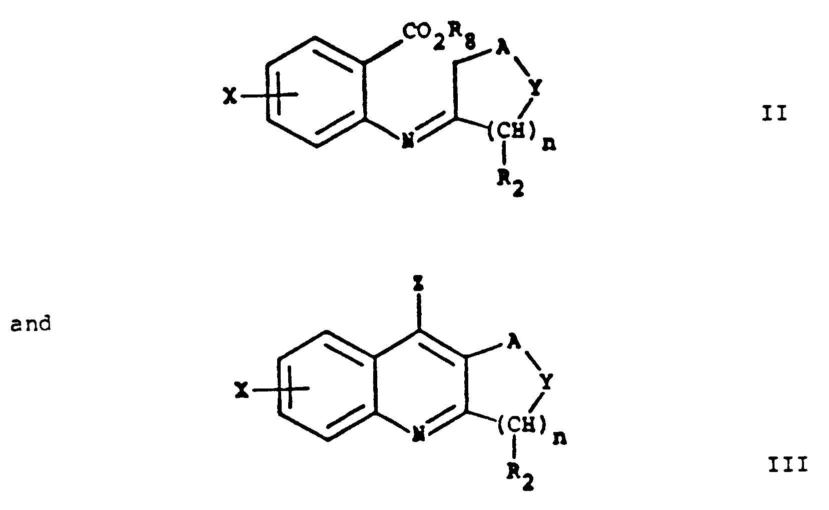
wherein A, X, Y, R₂ and n are as defined above, R₈ is hydrogen or C₁-C₆-alkyl, and Z is halogen, hydroxy or C₁-C₆-alkoxy, which are useful as intermediates for synthesizing the compounds of Formula I.

Throughout the specification and the appended claims, a given chemical formula or name shall encompass all stereo, optical and geometrical isomers thereof and tautomers where such isomers or tautomers exist, as well as pharmaceutically acceptable acid addition salts thereof and solvates thereof such as for instance hydrates.

Not included in the specification are compounds of formula I wherein A is CH₂, Y is NCH₃, (CHR₂)ₙ is CH₂CH₂, X is H, CH₃, Cl, Br or NO₂, R is H and R₁ is H, methyl, ethyl, propyl, butyl or benzyl which are disclosed in Khaldeeva and Konshin, Khim. Getrotsikl Soedin. 1976, No. 2, pp 263-265; those wherein A is -CH₂- or -CHR'-, Y is NH or NR', (CHR₂)ₙ is -CH₂CH₂- or -CH₂CHR'- and -NRR₁ is -NH₂, -NHC₆H₅ or di-C₁-C₆-alkylamino-C₁-C₆-alkylamino, each R' being independently C₁-C₆-alkyl which are disclosed in Wolf, U.S. Patents 3,580̸,915, 3,637,70̸6, 3,647,80̸0̸ and 3,674,790̸; those wherein A is CH₂, Y is NH or NR', (CHR₂)ₙ is -(CH₂)₃- or -CHR'CH₂CH₂- and -NRR₁ is -NH₂ and those wherein A is (CH₂)₂, Y is NH or NR', (CHR₂)ₙ is -CH₂CH₂- or -CHR'CH₂- and -NRR₁ is -NH₂ which are disclosed in Griss et al. U. S. Patent 3,987,0̸47.

The following definitions shall apply throughout the specification and the appended claims.

Unless otherwise stated or indicated, the term C₁-C₆-alkyl denotes a straight or branched alkyl group having from 1 to 6 carbon atoms. Examples of said alkyl include methyl, ethyl, n-propyl, iso-butyl, pentyl, and hexyl.

Unless otherwise stated or indicated, the term C₁-C₆-alkoxy denotes a straight or branched alkoxy group having 1 to 6 carbon atoms. Examples of said C₁-C₆-alkoxy include methoxy, ethoxy, iso-propoxy, sec-butoxy, and straight and branched chain hexyloxy.

Unless otherwise stated or indicated, the term halogen shall mean fluorine, chlorine, bromine or iodine.

Unless otherwise stated or indicated, the term oxygen-bridged shall signify the fact than an oxygen atom is present between phenyl and C₁-C₆-alkyl groups and/or an oxygen atom has replaced a methylene group in the C₁-C₆-alkyl group, with the proviso that said methylene group is not alpha to the amino nitrogen carrying the groups R and R₁. Thus, for instance, examples of oxygen-bridged phenyl-C₁-C₆-alkyl include 3-phenoxypropyl and 4-phenoxybutyl, and examples of oxygen-bridged di-phenyl-C₁-C₆-alkyl include 2-[bis(4-fluorophenyl)methoxy]ethyl and 2-[bis(3-fluorophenyl)methoxy]ethyl.

The compounds of this invention can be prepared by utilizing one or more of the steps described below.

In order to simplify the description of the synthetic schemes, the description will be presented with specific reference to the situation where A is CH₂, Y is oxygen, R₂ is hydrogen and n is 2, but it will readily be understood that the synthetic schemes can also be applied to the other situations by making obvious modifications where necessary.

Throughout the description of the synthetic steps, the definitions of X, R, R₁ and R₈ are as given above unless otherwise stated or indicated.

### STEP A

A compound of formula IIa can be prepared by reacting a compound of Formula IV with tetrahydro-4H-pyran-4-one. Said reaction can be conducted in a suitable solvent such as benzene, toluene or xylene at a temperature of about 80̸-150̸°C in the presence of an acid catalyst such as p-toluene sulfonic acid, benzenesulfonic acid or methanesulfonic acid.

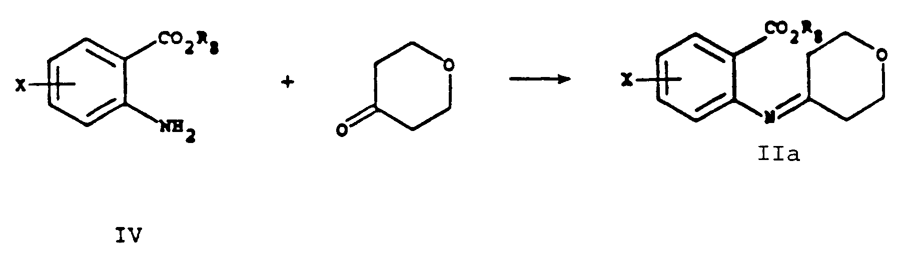

### STEP B

A compound of Formula IIIa can be prepared by reacting compound IIa with phosphorous pentoxide in the presence of a high boiling tertiary amine such as N,N-dimethylcyclohexylamine. Said reaction can be conducted without additional solvent at a temperature of about 170̸-220̸°C.

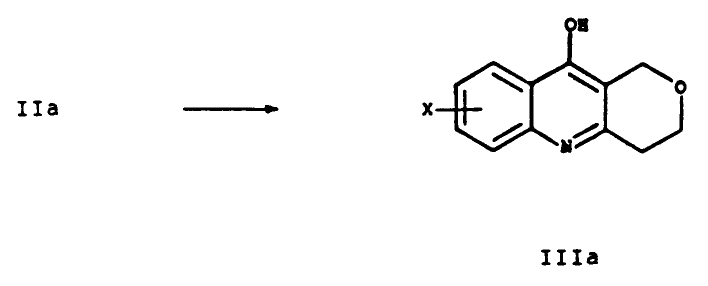

### STEP C

A compound of Formula IIIb can be prepared by reacting compound IIIa with phosphorous oxychloride and phosphorous pentachloride. Said reaction can be conducted at a temperature of about 10̸0̸-150̸°C.

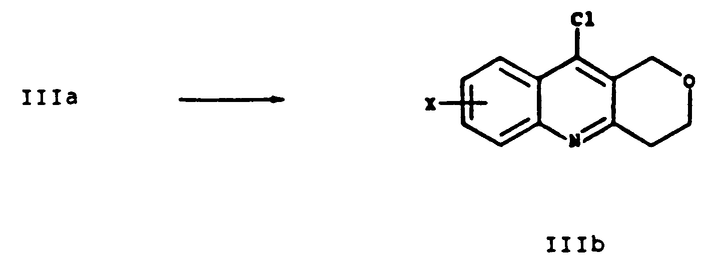

The bromine analogue of compound IIIb can be prepared in a similar manner, namely, for instance by reacting compound IIIa with phosphorous oxybromide and phosphorous pentabromide. The fluorine and iodine analogues of compound IIIa can be prepared by replacing the chlorine atom of compound IIIa with fluorine or iodine according to routine procedures known to the art.

### STEP D

A compound of Formula Ia can be prepared by reacting compound IIIb with an amine of formula V. Said reaction can be conducted at a temperature of 120̸-220̸°C in the presence of a hydroxylated aromatic compound such as phenol or cresol. 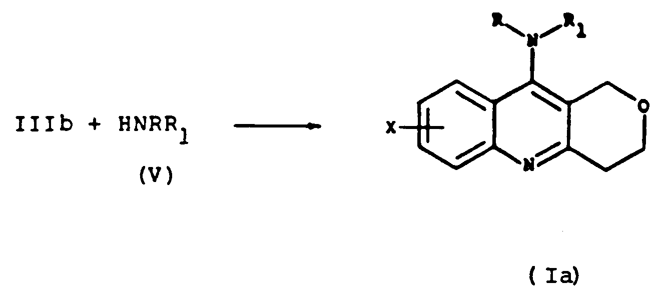
Steps A through D can be combined into a single step. Thus compound Ia can be obtained by heating together a mixture of phosphorous pentoxide, N,N-dimethylcyclohexylamine and the hydrochloride of amine V and then adding compound IV, followed by tetrahydro-4H-pyran-4-one. Said reaction can be carried out at a temperature of 150̸-250̸°C.

### STEP E

A compound of Formula Ib can be prepared by reacting an anthranilonitrile of Formula VII with tetrahydro-4H-pyran-4-one. Said reaction can be conducted in the presence of a Lewis acid such as zinc chloride, without solvent at a temperature of about 80̸-150̸°C or in a cosolvent such as 1,2-dichloroethane or nitrobenzene, again at a temperature of about 80̸-150̸°C. 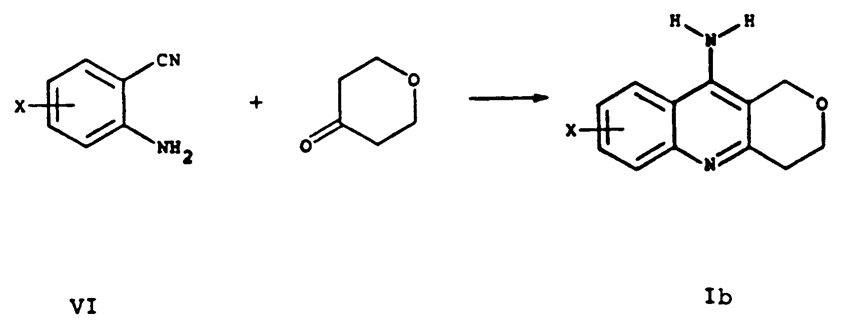
The compounds of Formula I of the present invention can be used for the treatment of various memory dysfunctions characterized by decreased cholinergic function, such as Alzheimer's disease.

This utility can be ascertained by determining the ability of these compounds to inhibit the activity of the enzyme acetylcholinesterase and thereby increase the acetylcholine levels in the brain.

This utility can also be ascertained by determining the ability of these compounds to restore cholinergically deficient memory in the Dark Avoidance Assay. In this assay mice are tested for their ability to remember an unpleasant stimulus for a period of 24 hours. A mouse is placed in a chamber that contains a dark compartment; a strong incandescent light drives it to the dark compartment, where an electric shock is administered through metal plates on the floor. The animal is removed from the testing apparatus and tested again, 24 hours later, for the ability to remember the electric shock.

If scopolamine, an anticholinergic that is known to cause memory impairment, is administered before an animal's initial exposure to the test chamber, the animal re-enters the dark compartment shortly after being placed in the test chamber 24 hours later. This effect of scopolamine is blocked by an active test compound, resulting in a greater interval before re-entry into the dark compartment.

Effective quantities of the compounds of the invention may be administered to a patient by any of the various methods, for example, orally as in capsules or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0̸.5% of active compound, but may be varied depending upon the particular form and may conveniently be between 4% to about 70̸% of the weight of the unit. The amount of active compound in such compositions is such that a suitable dosage will be obtained. Preferred compositions and preparations according to the present invention are prepared so that an oral dosage unit form contains between 1.0̸-30̸0̸ milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0̸.1% of active compound, but may be varied between 0̸.5 and about 30̸% of the weight thereof. The amount of active compound in such compositions is such that a suitable dosage will be obtained. Preferred compositions and preparations according to the present invention are prepared so that a parenteral dosage unit contains between 0̸.5 to 10̸0̸ milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparations can be enclosed in disposable syringes or multiple dose vials made of glass or plastic.

Examples of the compounds of this invention include:
9-amino-2,3-dihydrofuro[3,2-b]quinoline;
2,3-dihydro-9-methylaminofuro[3,2-b]quinoline;
9-benzylamino-2,3-dihydrothieno[3,2-b]quinoline;
9-(4-chloroanilino)-2,3-dihydrothieno[3,2-b]quinoline;
9-amino-1,3-dihydro-6-trifluoromethylthieno[3,4-b]quinoline;
9-anilino-1,3-dihydrothieno[3,4-b]quinoline;
10̸-amino-3,4-dihydro-2H-pyrano[3,2-b]quinoline;
9-amino-2,3-dihydrothieno[3,2-b]quinoline;
9-amino-1,3-dihydrothieno[3,4-b]quinoline;
3,4-dihydro-10̸-(4-fluorobenzylamino)-2H-pyrano[3,2-b]quinoline;
10̸-amino-3,4-dihydro-1H-pyrano[4,3-b]quinoline;
3,4-dihydro-10̸-propylamino-1H-pyrano[4,3-b]quinoline;
5-amino-7-chloro-3,4-dihydro-1H-pyrano[3,4-b]quinoline;
3,4-dihydro-5-[(4-methoxybenzyl)amino]-1H-pyrano[3,4-b]quinoline;
10̸-amino-3,4-dihydro-8-methoxy-1H-thiopyrano[3,2-b]quinoline;
10̸-(4-chlorobenzylamino)-3,4-dihydro-2H-thiopyrano[3,2-b]quinoline;
10̸-amino-3,4-dihydro-1H-thiopyrano[4,3-b]quinoline;
10̸-anilino-3,4-dihydro-1H-thiopyrano[4,3-b]quinoline;
5-amino-3,4-dihydro-8-trifluoromethyl-1H-thiopyrano[3,4-b]quinoline;
5-anilino-3,4-dihdyro-1H-thiopyrano[3,4-b]quinoline;
10̸-amino-1,3-dihydro-1,3-ethanofuro[3,4-b]quinoline;
10̸-benzylamino-1,3-dihydro-1,3-ethanothieno[3,4-b]quinoline;
9-amino-2,3-dihydro-1H-pyrrolo[3,2-b]quinoline;
9-amino-1,3-dihydro-2H-pyrrolo[3,4-b]quinoline;
10̸-amino-1,2,3,4-tetrahydrobenzo[b][1,5]naphthyridine;
5-amino-1,2,3,4-tetrahydrobenzo[b][1,3]naphthyridine;
11-amino-2,3,4,5-tetrahydro-1H-azepino[3,2-b]quinoline;
6-amino-2,3,4,5-tetrahydro-1H-azepino[3,4-b]quinoline;
10̸-amino-1,4-ethano-1,2,3,4-tetrahydrobenzo[b][1,5]naphthyridine;
10̸-amino-1,4-methano-1,2,3,4-terahydrobenzo[b][1,5]naphthyridine;
10̸-amino-1,3-ethano-1,2,3,4-tetrahydrobenzo[b][1,6]naphthyridine;
10̸-amino-1,4-ethano-1,2,3,4-tetrahydrobenzo[b][1,6]naphthyridine;
5-amino-1,3-ethano-1,2,3,4-tetrahydrobenzo[b][1,3]naphthyridine; and
5-amino-1,4-ethano-1,2,3,4-tetrahydrobenzo[b][1,3]naphthyridine.

The following examples are presented in order to illustrate this invention.

### EXAMPLE 1

### 10̸-Amino-3,4-dihydro-1H-pyrano[4,3-b]quinoline fumarate

Anthranilonitrile (5.91 g) was mixed with tetrahydro-4H-pyran-4-one (10̸.0̸ g) and then freshly fused ZnCl₂ (10̸.2 g) was added. The reaction mixture was heated at 120̸° for 3 hours and then it was distributed between 10̸% NaOH solution and 2-butanone. The organic phase was separated, dried and concentrated and the resulting residue was triturated with EtOAc (ethyl acetate) to remove some color. This material was further purified by flash chromatography (CH₂Cl₂, then 5% Et₃N-EtOAc). Concentation of the product-containing fractions and recrystallization from CH₂Cl₂-pentane gave 3.42 g of solid, mp 198-20̸0̸°C. The fumarate was formed in i-PrOH and recrystallized from H₂O to give analytically pure material, mp 258°(d).

| ANALYSIS: | | | |
|---|---|---|---|
| Calculated for C₁₂H₁₂N₂O·C₄H₄O₄: | 60̸.75%C | 5.10̸%H | 8.86%N |
| Found: | 60̸.42%C | 5.0̸9%H | 8.88%N |

### EXAMPLE 2

### 9-Amino-2,3-dihydrothieno[3,2-b]quinoline

Anthranilonitrile (4.80̸ g) and tetrahydrothiophen-3-one (8.17 g) were stirred until a homogeneous mixture was obtained and then freshly fused ZnCl₂ (8.0̸ g) was added and the reaction mixture heated at 120̸°. After 2 hours, 30̸ ml of 1,2-dichloroethane was added and the reaction mixture refluxed for an additional 2 hours. At the end of this time the reaction mixture was distributed between 10̸% NaOH solution and 2-butanone, after which the organic phase was separated, dried, concentrated and purified by flash chromatography (5% i-PrOH/CH₂Cl₂). Combination of the product-containing fractions gave 2.61 g of product that was pure to thin layer chromatography. This product was combined with that obtained in another run and recrystallized from EtOAc to give analytically pure material, mp 210̸-212°.

| ANALYSIS: | | | |
|---|---|---|---|
| Calculated for C₁₁H_{10̸}N₂S: | 65.31%C | 4.98%H | 13.85%N |
| Found: | 65.28%C | 4.98%H | 13.79%N |

### EXAMPLE 3

### 9-Amino-1,3-dihydrothieno[3,4-b]quinoline

Anthranilonitrile (4.80̸ g) and tetrahydrothiophen-3-one (8.17 g) were stirred until a homogeneous mixture was obtained and then fresly fused ZnCl₂ (8.0̸ g) was added and the reaction mixture heated at 120̸°. After 2 hours, 30̸ ml of 1,2-dichloroethane was added and the reaction mixture refluxed for an additional 2 hours. At the end of this time the reaction mixture was distributed between 10̸% NaOH solution and 2-butanone, after which the organic phase was separated, dried, concentrated and purified by flash chromatography (5% i-PrOH/CH₂Cl₂). Combination of the product-containing fractions gave 1.19 g of product that was pure to thin layer chromatography. This product was combined with that obtained in another run and recrystallized from EtOAc-pentane to give analytically pure material, mp 220̸°(d).

| ANALYSIS: | | | |
|---|---|---|---|
| Calculated for C₁₁H_{10̸}N₂S: | 65.31%C | 4.98%H | 13.85%N |
| Found: | 65.45%C | 4.95%H | 13.90̸%N |

### EXAMPLE 4

### 10̸-Amino-3,4-dihydro-1H-thiopyrano[4,3-b]quinoline

Tetrahydrothiopyran-4-one (10̸.0̸ g) was mixed with anthranilonitrile (5.0̸8 g) and the mixture warmed at 60̸° until a homogeneous solution was obtained. Freshly fused ZnCl₂ (8.2 g) was then added portionwise and the temperature of the reaction mixture raised to 120̸°. After 2 hours it was cooled and distributed between 10̸% NaOH and 2-butanone. The organic phase was separated, dried, and concentrated and the crude product triturated with Et₂O and then passed over a silica gel column (5% Et₃N-ethyl acetate). The product-containing fractions were concentrated and the product recrystallized from toluene to give 3.66 g, mp 214-216°.

| ANALYSIS: | | | |
|---|---|---|---|
| Calculated for C₁₂H₁₂N₂S: | 66.63%C | 5.59%H | 12.95%N |
| Found: | 66.74%C | 5.71%H | 12.79%N |

### EXAMPLE 5

### 10-amino-3,4-dihydro-7-methyl-1H-thiopyrano[4,3-b]quinoline

A mixture of 4-methylanthranilonitrile (4.46 g) and freshly fused zinc chloride (6.8 g) in 20 ml of nitrobenzene was heated at 50°C for 1 hour. To this was added tetrahydrothiopyran-4-one (5.1 g) and the mixture was heated to 130°C for 1.5 hours. After cooling, the zinc complex was filtered, rinsed with ethyl ether and partitioned between 2-butanone (MEK) and NH₄OH. The aqueous phase was extracted with MEK and the organics were washed with water and dried (saturated NaCl, MgSO₄). This was concentrated to give 4.76 g of an off-white powder, m.p. 239-245°C d., which was recrystallized from ethyl acetate to give 2.95 g of an off-white powder, m.p. 243-246°C d.

| ANALYSIS: | | | |
|---|---|---|---|
| Calculated for C₁₃H₁₄N₂S: | 67.79%C | 6.13%H | 12.16%N |
| Found: | 67.67%C | 6.12%H | 12.14%N |

### EXAMPLE 6

### 10-amino-3,4-dihydro-7-fluoro-1H-thiopyrano[4,3-b]quinoline

A mixture of 4-fluoroanthranilonitrile (4.47 g) and freshly fused, pulverized ZnCl₂ (6.7 g) in 20 ml of nitrobenzene was heated at 50°C for 45 minutes. To the resulting suspension was added thiopyran-2-one (5.1 g). This was heated at 130°C for 3 hours. The resulting precipitate was filtered, rinsed with ether and partioned between 2-butanone and NH₄OH solution. The aqueous layer was extracted with 2-butanone and the combined organics were washed with water and dried (MgSO₄). This resulted in 4.8 g of a tan solid, m.p. 234-237°C d., which was recrystallized from ethyl acetate to give 3.0 g of a white powder, m.p. 235-237°C d.

| ANALYSIS: | | | |
|---|---|---|---|
| Calculated for C₁₂H₁₁FN₂S: | 61.51%C | 4.73%H | 11.96%N |
| Found: | 61.48%C | 4.70%H | 11.93%N |

### Example 7

### 10-amino-3,4-dihydro-7-trifluoromethyl-1H-thiopyrano[4,3-b]quinoline

To a solution of 4-trifluoromethyl anthranilonitrile (12.2 g) in 50 ml of nitrobenzene was added freshly fused and pulverized zinc chloride (13.4 g). This was stirred at 50°C for 1.5 hours after which was added tetrahydrothiopyran-4-one (9.9 g). The reaction mixture was stirred at 130°C for 2 hours and allowed to cool, and the resulting complex was filterd and rinsed with ethyl ether. The solid was partioned between 2-butanone (MEK) and NH₄OH and the aqueous phase was extracted with MEK (2x). The organics were washed with water, dried (saturated NaCl, MgSO₄) and concentrated to give 10.2 g of a yellow powder m.p. 253-259°C d. A 3.7 g portion was recrystallized from methanol/water to give 3.0 g of an off-white powder; m.p. 258-262°C d.

| ANALYSIS: | | | |
|---|---|---|---|
| Calculated for C₁₃H₁₁F₃N₂S: | 54.92%C | 3.90%H | 9.85%N |
| Found: | 54.91%C | 4.06%H | 9.82%N |

### EXAMPLE 8

### N-(Phenylmethylene)-3,4-dihydrothiopyrano-1H-[4,3-b]quinolin-10-amine

3,4-Dihydrothiopyrano-1H-[4,3-b]quinolin-10-amine (8.64 g) was suspended in 300 ml of toluene to which morpholine (7.0 g) and benzaldehyde (8.50 g, freshly washed with aqueous K₂CO₃ solution) were then added. This reaction mixture was then refluxed overnight with the separation of H₂O (Dean-Stark trap) and then concentrated and purified by flash chromatography (20 % ethyl acetate/CH₂Cl₂). The product-containing fractions were concentrated to give 7.30 g of chromatographically pure product, m.p. 171-173°C. Analytically pure material was obtained by recrystallization from CH₂Cl₂/pentane, m.p. 175-176°C.

| ANALYSIS: | | | |
|---|---|---|---|
| Calculated for C₁₉H₁₆N₂S: | 74.96%C | 5.30%H | 9.20%N |
| Found: | 74.97%C | 5.25%H | 9.18%N |

### EXAMPLE 9

### 10-benzylamino-3,4-dihydro-1H-thiopyrano[4,3-b]quinoline hydrochloride

N-(Phenylmethylene)-3,4-dihydrothiopyrano-1H-[4,3-b] quinolin-10-amine (3.51 g) was dissolved in 30 ml of HOAc and 0.94 g of NaCNBH₃ was added portionwise. After 30 minutes the solvent was evaporated and the residue distributed between 2-butanone and NaHCO₃ solution. The organic phase was separated, dried and concentrated to give crude product which was then shaken with Et₂O and 5 % HCl. The insoluble hydrochloride was filtered and recrystallized from EtOH/Et₂O to give 2.15 g, m.p. 270°C (d).

| ANALYSIS: | | | |
|---|---|---|---|
| Calculated for C₁₉H₁₈N₂S·HCl: | 66.55%C | 5.59%H | 8.17%N |
| Found: | 66.13%C | 5.57%H | 8.03%N |

## Claims (Claims for the following Contracting State(s): AT, BE, CH, DE, FR, GB, IT, LI, LU, NL, SE)

1. A compound of the formula I 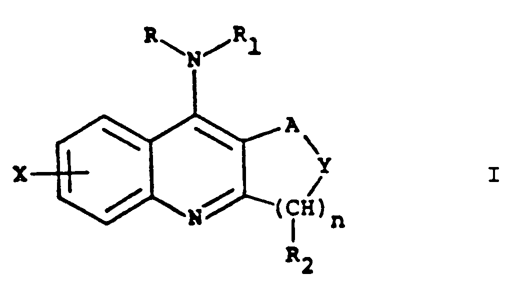 wherein n is 1-4; R is hydrogen, C₁-C₆-alkyl or C₁-C₆-alkylcarbonyl; R₁ is hydrogen, C₁-C₆-alkyl, C₁-C₆-alkylcarbonyl, di-C₁-C₆-alkylamino-C₁-C₆-alkyl or phenyl, phenyl-C₁-C₆-alkyl, diphenyl-C₁-C₆-alkyl oxygen-bridged phenyl-C₁-C₆-alkyl, or oxygen-bridged diphenyl-C₁-C₆-alkyl where the phenyl may be substituted with 1, 2 or 3 substituents each of which being independently C₁-C₆-alkyl, C₁-C₆-alkoxy, halogen, hydroxy, trifluoromethyl, phenoxy or benzyloxy; A is a A is a direct bond or (CHR₃)ₘ, m being 1-3; X is hydrogen, C₁-C₆-alkyl, C₃-C₇-cycloalkyl, C₁-C₆-alkoxy, halogen, hydroxy, nitro, trifluoromethyl, formyl, C₁-C₆-alkylcarbonyl, phenylcarbonyl where the phenyl may be substituted with 1, 2 or 3 substituents each of which being independently C₁-C₆-alkyl, C₁-C₆-alkoxy, halogen, hydroxy, trifluoromethyl, phenoxy or benzyloxy, -SH, C₁-C₆-alkylthio, -NHCOR₄ or -NR₅R₆, R₄ being hydrogen or C₁-C₆-alkyl, and R₅ and R₆ being independently hydrogen, C₁-C₆-alkyl or C₃-C₇-cycloalkyl; Y is O, S or NR₇; and each R₂, each R₃ and R₇ are independently hydrogen or C₁-C₆-alkyl, or taken two at a time form a methylene or ethylene group constituting a part of a ring of at least five atoms; with the proviso that when A is CH₂, Y is NCH₃, (CHR₂)ₙ is CH₂CH₂, X is H, CH₃, Cl, Br or NO₂ and R is H, R₁ is not H, methyl, ethyl, propyl, butyl or benzyl, that when A is -CH₂- or -CHR'-, Y is NH or NR' and (CHR₂)ₙ is -CH₂CH₂- or -CH₂CHR'-, the group -NRR₁ is not -NH₂, -NHC₆H₅ or di-C₁-C₆-alkylamino-C₁-C₆-alkylamino, each R' being independently C₁-C₆-alkyl, that when A is CH₂, Y is NH or NR' and (CHR₂)ₙ is -(CH₂)₃- or -CHR'CH₂CH₂-, the group -NRR₁ is not -NH₂ and that when A is -CH₂CH₂-, Y is NH or NR' and (CHR₂)ₙ is -CH₂CH₂- or -CHR'CH₂-, the group -NRR₁ is not -NH₂; stereo, optical and geometrical isomers thereof, or a pharmaceutically acceptable acid addition salt thereof.

2. The compound as defined in Claim 1, where A is CH₂, Y is O or S, R is hydrogen, R₁ is hydrogen or phenyl-C₁-C₆-alkyl wherein the phenyl may be substituted as defined in claim 1, R₂ is hydrogen and n is 2.

3. The compound as defined in Claim 2, where X is hydrogen, C₁-C₆-alkyl or trifluoromethyl.

4. The compound as defined in Claim 1, which is 9-amino-2,3-dihydrothieno[3,2-b]quinoline.

5. The compound as defined in Claim 1, which is 9-amino-1,3-dihydrothieno[3,4-b]quinoline.

6. The compound as defined in Claim 1, which is 10-amino-3,4-dihydro-1H-pyrano[4,3-b]quinoline.

7. The compound as defined in Claim 1, which is 10-amino-3,4-dihydro-1H-thiopyrano[4,3-b]quinoline.

8. A process for the preparation of a compound as defined in claim 1, which comprises
a) reacting a compound of the formula III 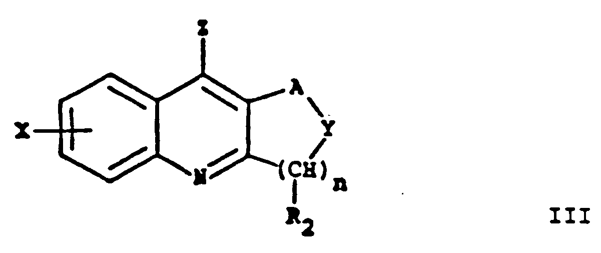 where A, Y, R₂ and n are as defined and Z is halogen, with an amine of the formula V
HNRR₁ V
where R and R₁ are as defined, to afford a compound of the formula I, or
b) heating a compound of the formula IV 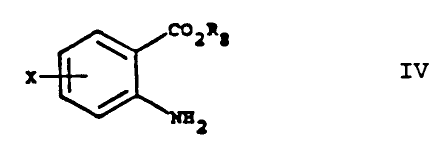 where R₈ is hydrogen or loweralkyl, with a mixture of phosphorous pentoxide, N,N-dimethylcyclohexylamine and the hydrochloride of an amine of the formula HNRR₁, where R and R₁ are as defined, and adding tetrahydro-4H-pyran-4-one to afford a compound of the formula Ia, 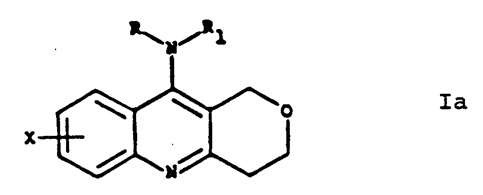 where R and R₁ are as defined, or
c) reacting a compound of formula VI 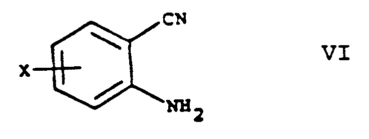 where X is as defined, with tetrahydro-4H-pyran-4-one to afford a compound of the formula Ib

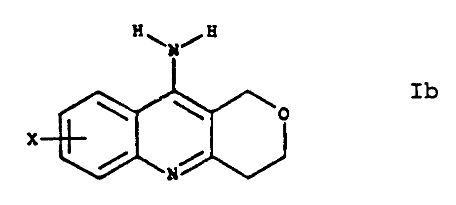

9. A pharmaceutical composition which comprises as the active ingredients a compound as defined in claim 1 and a pharmaceutically acceptable carrier.

10. Use of a compound as defined in claim 1 for the preparation of a medicament having the activity of increasing the cholinergic function in mammals.

11. A compound having the formula II 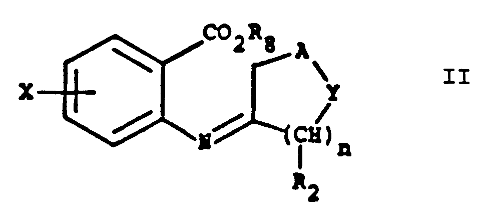 wherein n, A, X and Y are as defined in claim 1, R₈ is hydrogen or C₁-C₆-alkyl; and R₂, R₃ and R₇ are independently hydrogen or C₁-C₆-alkyl, or taken two at a time form a methylene or ethylene group constituting a part of a ring of at least five atoms.

12. A compound having the formula III 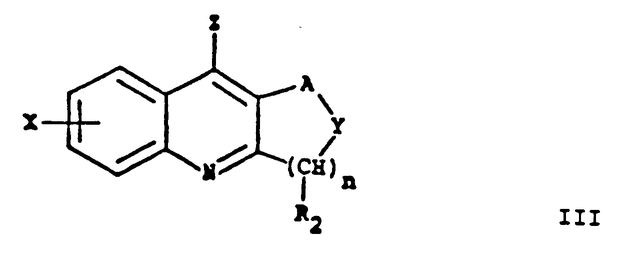 wherein n, A, X and Y are as defined in claim 1, Z is halogen, hydroxy or C₁-C₆-alkoxy; and R₂, R₃ and R₇ are independently hydrogen or C₁-C₆-alkyl, or taken two at a time form a methylene or ethylene group constituting a part of a ring of at least five atoms with the proviso that when A is CH₂, Y is NCH₃, (CHR₂)ₙ, is CH₂CH₂ and X is H, CH₃, Cl, Br or NO₂, Z is not Cl.

## Claims (Claims for the following Contracting State(s): ES, GR)

1. A process for the preparation of a compound of the formula I 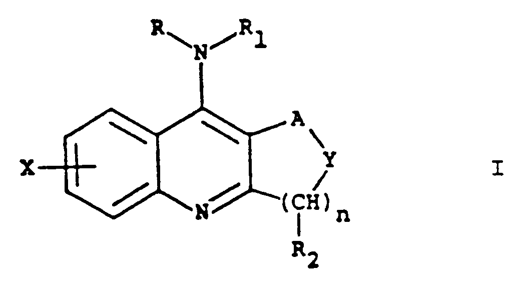 wherein n is 1-4; R is hydrogen, C₆-C₆-alkyl or C₁-C₆-alkylcarbonyl; R₁ is hydrogen, C₁-C₆-alkyl, C₁-C₆-alkylcarbonyl, di(C₁-C₆-alkyl)amino-C₁-C₆-alkyl or phenyl, phenyl-C₁-C₆-alkyl, diphenyl-C₁-C₆-alkyl, oxygen-bridged phenyl-C₁-C₆-alkyl, or oxygen-bridged diphenyl-C₁-C₆-alkyl where the phenyl may be substituted with 1, 2 or 3 substituents each of which being independently C₁-C₆-alkyl, C₁-C₆-alkoxy, halogen, hydroxy, trifluoromethyl, phenoxy or benzyloxy; A is a direct bond or (CHR₃)ₘ, m being 1-3; X is hydrogen, C₁-C₆-alkyl, C₃-C₇-cycloalkyl, C₁-C₆-alkoxy, halogen, hydroxy, nitro, trifluoromethyl, formyl, C₁-C₆-alkylcarbonyl, phenylcarbonyl wherein the phenyl my be substituted as indicated above, -SH, C₁-C₆-alkylthio, -NHCOR₄ or -NR₅R₆, R₄ being hydrogen or C₁-C₆-alkyl, and R₅ and R₆ being independently hydrogen, C₁-C₆-alkyl or C₃-C₇-cycloalkyl; Y is O, S or NR₇; and each R₂, each R₃ and R₇ are independently hydrogen or C₁-C₆-alkyl, or taken two at a time form a methylene or ethylene group constituting a part of a ring of at least five atoms; with the proviso that when A is CH₂, Y is NCH₃, (CHR₂)ₙ is CH₂CH₂, X is H, CH₃, Cl, Br or NO₂ and R is H, R₁ is not H, methyl, ethyl, propyl, butyl or benzyl, that when A is -CH₂- or -CHR'-, Y is NH or NR' and (CHR₂)ₙ is -CH₂CH₂- or -CH₂CHR'-, the group -NRR₁ is not -NH₂, -NHC₆H₅ or di-C₁-C₆-alkylamino-C₁-C₆-alkylamino, each R' being independently C₁-C₆-alkyl, that when A is CH₂, Y is NH or NR' and (CHR₂)ₙ is -(CH₂)₃- or -CHR'CH₂CH₂-, the group -NRR₁ is not -NH₂ and that when A is -CH₂CH₂-, Y is NH or NR' and (CHR₂)ₙ is -CH₂CH₂- or -CHR'CH₂-, the group -NRR₁ is not -NH₂; stereo, optical and geometrical isomers thereof, or a pharmaceutically acceptable acid addition salt thereof, which comprises
a) reacting a compound of the formula III 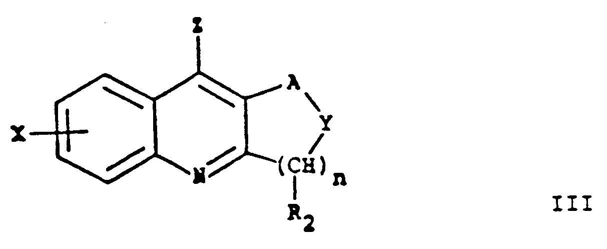 where A, Y, R₂ and n are as defined and Z is halogen, with an amine of the formula V
HNRR₁ V
where R and R₁ are as defined, to afford a compound of the formula I, or
b) heating a compound of the formula IV 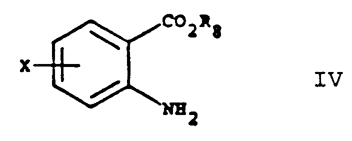 where R₈ is hydrogen or loweralkyl, with a mixture of phosphorous pentoxide, N,N-dimethylcyclohexylamine and the hydrochloride of an amine of the formula HNRR₁, where R and R₁ are as defined, and adding tetrahydro-4H-pyran-4-one to afford a compound of the formula Ia, 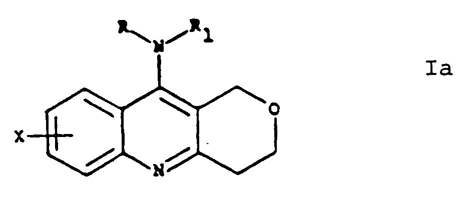 where R and R₁ are as defined, or
c) reacting a compound of formula VI 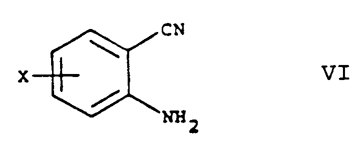 where X is as defined, with tetrahydro-4H-pyran-4-one to afford a compound of the formula Ib

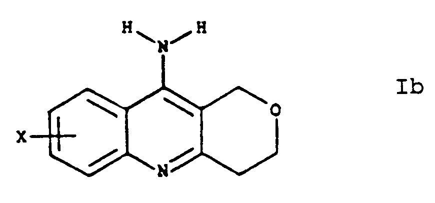

2. The compound as defined in Claim 1, where A is CH₂, Y is O or S, R is hydrogen, R₁ is hydrogen or phenyl-C₁-C₆-alkyl wherein the phenyl may be substituted as defined in claim 1, R₂ is hydrogen and n is 2.

3. A process as defined in claim 2, where X is hydrogen, loweralkyl or trifluoromethyl.

4. A process as defined in claim 1, where 9-amino-2,3-dihydrothieno[3,2-b]quinoline is prepared.

5. A process as defined in claim 1, where 9-amino-1,3-dihydrothieno[3,4-b]quinoline is prepared.

6. A process as defined in claim 1, where 10-amino-3,4-dihydro-1H-pyrano[4,3-b]quinoline is prepared.

7. A process as defined in claim 1, where 10-amino-3,4-dihydro-1H-thiopyrano[4,3-b]quinoline is prepared.

8. A process for the preparation of a compound of the formula II 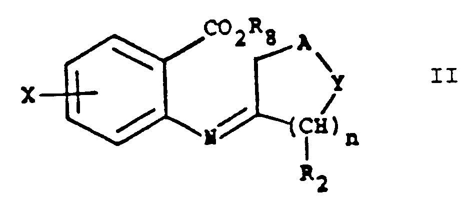 wherein n, A, X and Y are as defined in claim 1, R₈ is hydrogen or C₁-C₆-alkyl; and R₂, R₃ and R₇ are independently hydrogen or C₁-C₆-alkyl, or taken two at a time form a methylene or ethylene group constituting a part of a ring of at least five atoms, which comprises reacting a compound of the formula IV 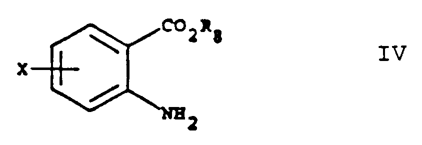 where X and R₈ are as defined above, with a compound of the formula VII 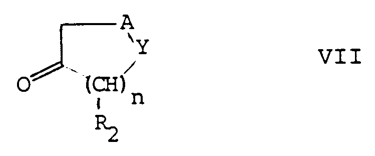 where A, Y, R₂ and n are as defined.

9. A process for the preparation of a compound of the formula III 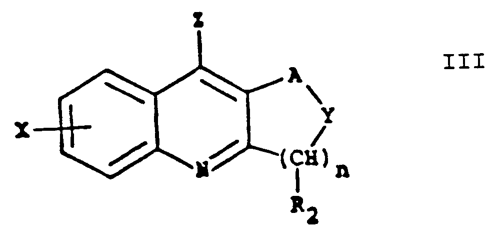 wherein n, A, X and Y are as defined in claim 1, Z is halogen, hydroxy or C₁-C₆-alkoxy; and R₂, R₃ and R₇ are independently hydrogen or C₁-C₆-alkyl, or taken two at a time form a methylene or ethylene group constituting a part of a ring of at least five atoms with the proviso that when A is CH₂, Y is NCH₃, (CHR₂)ₙ is CH₂CH₂ and X is H, CH₃, Cl, Br or NO₂, Z is not Cl, which comprises
a) cyclising a compound of the formula II 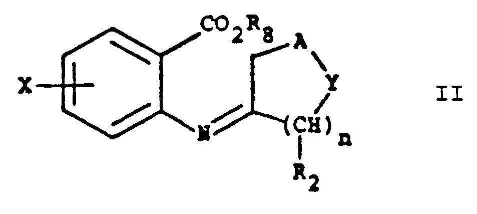 where A, Y, X, R₂, R₈ and n are as defined, in the presence of phosphorous pentoxide to afford a compound of the formula III where Z is hydroxy, and
b) optionally reacting a compound of the formula III where Z is hydroxy, with a mixture of POCl₃ and PCl₅ or POBr₃ and PBr₅ to afford a compound of the formula III where Z is Cl and Br, respectively, and
c) optionally converting a compound of the formula III where Z is chlorine, according to routine procedures known to the art to a compound of the formula III where Z is fluorine or iodine.

10. Use of a compound as defined in claim 1 for the preparation of a medicament having the activity of increasing the cholinergic function in mammals.

## Patentansprüche (Patentansprüche für folgende(n) Vertragsstaat(en): AT, BE, CH, DE, FR, GB, IT, LI, LU, NL, SE)

1. Eine Verbindung mit der Formel I 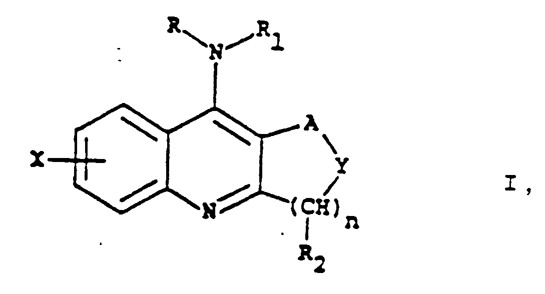 worin n 1 bis 4 ist; R für Wasserstoff, C₁-C₆-Alkyl oder C₁-C₆-Alkylcarbonyl steht; R₁ Wasserstoff, C₁-C₆-Alkyl, C₁-C₆-Alkylcarbonyl, Di-C₁-C₆-alkylamino-C₁-C₆-alkyl oder Phenyl, Phenyl-C₁-C₆-alkyl, Di-phenyl-C₁-C₆-alkyl, Sauerstoff-verbrücktes Phenyl-C₁-C₆-alkyl oder Sauerstoff-verbrücktes Di-phenyl-C₁-C₆-alkyl bedeutet, worin das Phenyl durch 1, 2 oder 3 Substituenten substituiert sein kann, die jeweils unabhängig voneinander C₁-C₆-Alkyl, C₁-C₆-Alkoxy, Halogen, Hydroxy, Trifluormethyl, Phenoxy oder Benzyloxy sind; A eine direkte Bindung oder (CHR₃)ₘ bedeutet, worin m 1 bis 3 ist; X für Wasserstoff, C₁-C₆-Alkyl, C₃-C₇-Cycloalkyl, C₁-C₆-Alkoxy, Halogen, Hydroxy, Nitro, Trifluormethyl, Formyl, C₁-C₆-Alkylcarbonyl, Phenylcarbonyl steht, worin das Phenyl durch 1, 2 oder 3 Substituenten substituiert sein kann, die jeweils unabhängig voneinander C₁-C₆-Alkyl, C₁-C₆-Alkoxy, Halogen, Hydroxy, Trifluormethyl, Phenoxy oder Benzyloxy, -SH, C₁-C₆-Alkylthio, -NHCOR₄ oder -NR₅R₆ sind, worin R₄ Wasserstoff oder C₁-C₆-Alkyl bedeutet und R₅ und R₆ unabhängig voneinander Wasserstoff, C₁-C₆-Alkyl oder C₃-C₇-Cycloalkyl bedeuten; Y für O, S oder NR₇ steht; und R₂, R₃ und R₇ jeweils unabhängig voneinander Wasserstoff oder C₁-C₆-Alkyl bedeuten, oder zwei dieser Reste gemeinsam eine Methylen- oder Ethylengruppe ausbilden, die einen Teil eines Ringes mit wenigstens fünf Atomen darstellt; mit der Maßgabe, daß dann, wenn A für CH₂ steht, Y für NCH₃ steht, (CHR₂)ₙ für CH₂CH₂ steht, X Wasserstoff, CH₃, Cl, Br oder NO₂ darstellt und R Wasserstoff bedeutet, R₁ nicht Wasserstoff, Methyl, Ethyl, Propyl, Butyl oder Benzyl darstellt, daß dann, wenn A für -CH₂- oder -CHR'- steht, Y für NH oder NR' steht und (CHR₂)ₙ für -CH₂CH₂- oder -CH₂CHR'- steht, die Gruppe -NRR₁ nicht -NH₂, -NHC₆H₅ oder Di-C₁-C₆-alkylamino-C₁-C₆-alkylamino darstellt, wobei jeder Rest R' unabhängig voneinander C₁-C₆-Alkyl bedeutet, daß dann, wenn A für CH₂ steht, Y für NH oder NR' steht und (CHR₂)ₙ für -(CH₂)₃- oder -CHR'CH₂CH₂- steht, die Gruppe -NRR₁ nicht -NH₂ bedeutet, und daß dann, wenn A für -CH₂CH₂- steht, Y für NH oder NR' steht und (CHR₂)ₙ für -CH₂CH₂- oder -CHR'CH₂- steht, die Gruppe -NRR₁ nicht -NH₂ bedeutet; die stereomeren, optischen und geometrischen Isomeren hievon oder ein pharmazeutisch annehmbares Säureadditionssalz hievon.

2. Verbindung nach Anspruch 1, worin A für CH₂ steht, Y die Bedeutung O oder S hat, R Wasserstoff ist, R₁ für Wasserstoff oder Phenyl-C₁-C₆-alkyl steht, worin das Phenyl wie in Anspruch 1 definiert substituiert sein kann, R₂ Wasserstoff bedeutet und n den Wert 2 hat.

3. Verbindung nach Anspruch 2, worin X Wasserstoff, C₁-C₆-Alkyl oder Trifluormethyl bedeutet.

4. Verbindung nach Anspruch 1, nämlich 9-Amino-2,3-dihydrothieno[3,2-b]chinolin.

5. Verbindung nach Anspruch 1, nämlich 9-Amino-1,3-dihydrothieno[3,4-b]chinolin.

6. Verbindung nach Anspruch 1, nämlich 10-Amino-3,4-dihydro-1H-pyrano[4,3-b]chinolin.

7. Verbindung nach Anspruch 1, nämlich 10-Amino-3,4-dihydro-1H-thiopyrano[4,3-b]chinolin.

8. Verfahren zur Herstellung einer Verbindung nach Anspruch 1, welches umfaßt:
a) Umsetzen einer Verbindung der Formel III 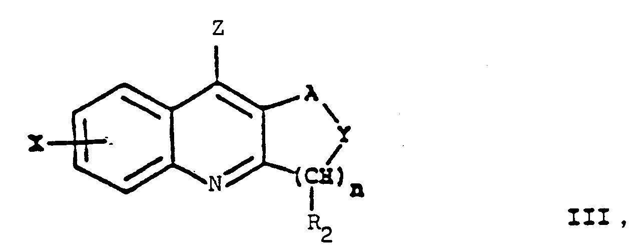 worin A, Y, R₂ und n wie definiert sind und Z Halogen bedeutet, mit einem Amin der Formel V
HNRR₁ V ,
worin R und R₁ wie oben definiert sind, zur Ausbildung einer Verbindung der Formel I, oder
b) Erhitzen einer Verbindung der Formel IV 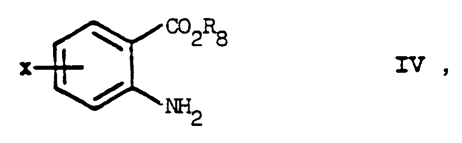 worin R₈ Wasserstoff oder Niederalkyl ist, mit einem Gemisch aus Phosphorpentoxid, N,N-Dimethylcyclohexylamin und dem Hydrochlorid eines Amins der Formel HNRR₁, worin R und R₁ wie oben definiert sind, und Zusetzen von Tetrahydro-4H-pyran-4-on zur Ausbildung einer Verbindung der Formel Ia 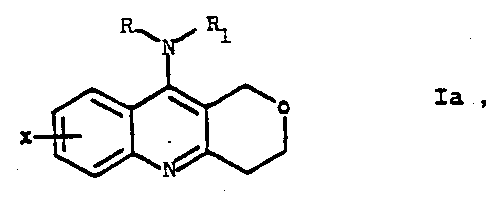 worin R und R₁ wie oben definiert sind, oder
c) Umsetzen einer Verbindung der Formel VI 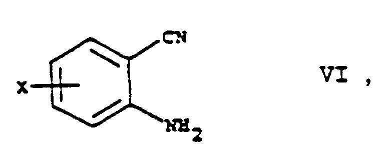 worin X wie oben definiert ist, mit Tetrahydro-4H-pyran-4-on zur Ausbildung einer Verbindung der Formel Ib

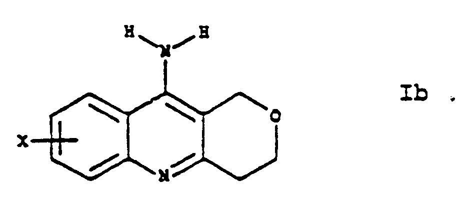

9. Pharmazeutische Zusammensetzung, die als wirksamen Bestandteil eine Verbindung gemäß Definition in Anspruch 1 und einen pharmazeutisch annehmbaren Träger umfaßt.

10. Verwendung einer Verbindung nach Anspruch 1 zur Herstellung eines Medikaments mit der Wirksamkeit der Erhöhung der cholinergen Funktionen in Säugetieren.

11. Eine Verbindung mit der Formel II 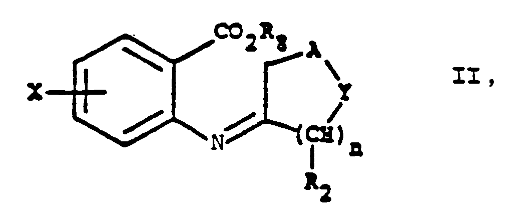 worin n, A, X und Y wie in Anspruch 1 definiert sind, R₈ Wasserstoff oder C₁-C₆-Alkyl bedeutet und R₂, R₃ und R₇ unabhängig voneinander Wasserstoff oder C₁-C₆-Alkyl darstellen, oder zwei dieser Reste gemeinsam eine Methylen- oder Ethylengruppe ausbilden, die einen Teil eines Ringes mit wenigstens fünf Atomen darstellt.

12. Verbindung mit der Formel III 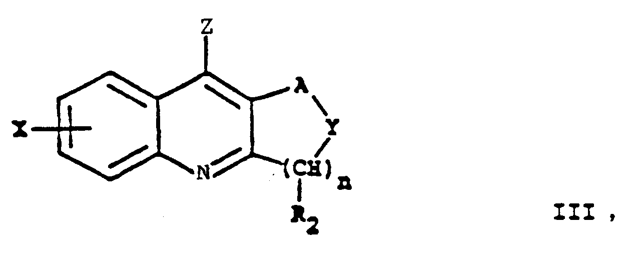 worin n, A, X und Y wie in Anspruch 1 definiert sind, Z für Halogen, Hydroxy oder C₁-C₆-Alkoxy steht; und R₂, R₃ und R₇ unabhängig voneinander Wasserstoff oder C₁-C₆-Alkyl darstellen, oder zwei dieser Reste gemeinsam eine Methylen- oder Ethylengruppe ausbilden, die einen Teil eines Ringes aus wenigstens fünf Atomen darstellen, mit der Maßgabe, daß dann, wenn A für CH₂ steht, Y für NCH₃ steht, (CHR₂)ₙ für CH₂CH₂ steht und X Wasserstoff, CH₃, Cl, Br oder NO₂ darstellt, Z nicht für Cl steht.

## Patentansprüche (Patentansprüche für folgende(n) Vertragsstaat(en): ES, GR)

1. Verfahren zur Herstellung einer Verbindung mit der Formel I 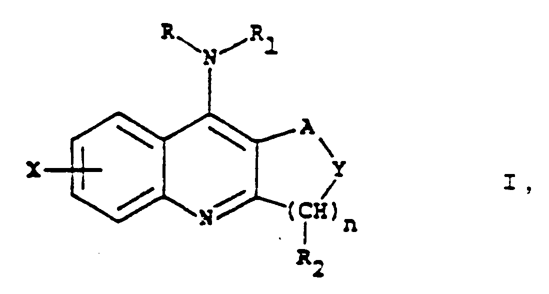 worin n 1 bis 4 ist; R für Wasserstoff, C₁-C₆-Alkyl oder C₁-C₆-Alkylcarbonyl steht; R₁ Wasserstoff, C₁-C₆-Alkyl, C₁-C₆-Alkylcarbonyl, Di-C₁-C₆-alkylamino-C₁-C₆-alkyl oder Phenyl, Phenyl-C₁-C₆-alkyl, Di-phenyl-C₁-C₆-alkyl, Sauerstoff-verbrücktes Phenyl-C₁-C₆-alkyl oder Sauerstoff-verbrücktes Di-phenyl-C₁-C₆-alkyl bedeutet, worin das Phenyl durch 1, 2 oder 3 Substituenten substituiert sein kann, die jeweils unabhängig voneinander C₁-C₆-Alkyl, C₁-C₆-Alkoxy, Halogen, Hydroxy, Trifluormethyl, Phenoxy oder Benzyloxy sind; A eine direkte Bindung oder (CHR₃)ₘ bedeutet, worin m 1 bis 3 ist; X für Wasserstoff, C₁-C₆-Alkyl, C₃-C₇-Cycloalkyl, C₁-C₆-Alkoxy, Halogen, Hydroxy, Nitro, Trifluormethyl, Formyl, C₁-C₆-Alkylcarbonyl, Phenylcarbonyl steht, worin das Phenyl durch 1, 2 oder 3 Substituenten substituiert sein kann, die jeweils unabhängig voneinander C₁-C₆-Alkyl, C₁-C₆-Alkoxy, Halogen, Hydroxy, Trifluormethyl, Phenoxy oder Benzyloxy, -SH, C₁-C₆-Alkylthio, -NHCOR₄ oder -NR₅R₆ sind, worin R₄ Wasserstoff oder C₁-C₆-Alkyl bedeutet und R₅ und R₆ unabhängig voneinander Wasserstoff, C₁-C₆-Alkyl oder C₃-C₇-Cycloalkyl bedeuten; Y für O, S oder NR₇ steht; und R₂, R₃ und R₇ jeweils unabhängig voneinander Wasserstoff oder C₁-C₆-Alkyl bedeuten, oder zwei dieser Reste gemeinsam eine Methylen- oder Ethylengruppe ausbilden, die einen Teil eines Ringes mit wenigstens fünf Atomen darstellt; mit der Maßgabe, daß dann, wenn A für CH₂ steht, Y für NCH₃ steht, (CHR₂)ₙ für CH₂CH₂ steht, X Wasserstoff, CH₃, Cl, Br oder NO₂ darstellt und R Wasserstoff bedeutet, R₁ nicht Wasserstoff, Methyl, Ethyl, Propyl, Butyl oder Benzyl darstellt, daß dann, wenn A für -CH₂- oder -CHR'- steht- Y für NH oder NR' steht und (CHR₂)ₙ für -CH₂CH₂- oder -CH₂CHR'- steht, die Gruppe -NRR₁ nicht -NH₂, -NHC₆H₅ oder Di-C₁-C₆-alkylamino-C₁-C₆-alkylamino darstellt, wobei jeder Rest R' unabhängig voneinander C₁-C₆-Alkyl bedeutet, daß dann, wenn A für CH₂ steht, Y für NH oder NR' steht und (CHR₂)ₙ für -(CH₂)₃- oder -CHR'CH₂CH₂- steht, die Gruppe -NRR₁ nicht -NH₂ bedeutet, und daß dann, wenn A für -CH₂CH₂- steht, Y für NH oder NR' steht und (CHR₂)ₙ für -CH₂CH₂- oder -CHR'CH₂- steht, die Gruppe -NRR₁ nicht -NH₂ bedeutet; von stereomeren, optischen und geometrischen Isomeren hievon oder eines pharmazeutisch annehmbaren Säureadditionssalzes hievon, welches umfaßt:
a) Umsetzen einer Verbindung der Formel III 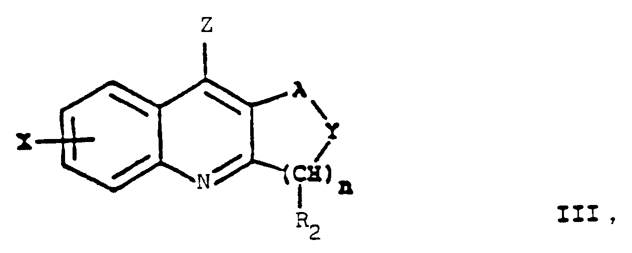 worin A, Y, R₂ und n wie definiert sind und Z Halogen bedeutet, mit einem Amin der Formel V
HNRR₁ V ,
worin R und R₁ wie oben definiert sind, zur Ausbildung einer Verbindung der Formel I, oder
b) Erhitzen einer Verbindung der Formel IV 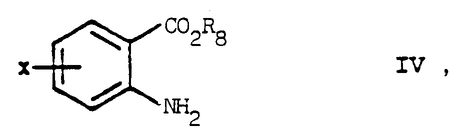 worin R₈ Wasserstoff oder Niederalkyl ist, mit einem Gemisch aus Phosphorpentoxid, N,N-Dimethylcyclohexylamin und dem Hydrochlorid eines Amins der Formel HNRR₁, worin R und R₁ wie oben definiert sind, und Zusetzen von Tetrahydro-4H-pyran-4-on zur Ausbildung einer Verbindung der Formel Ia 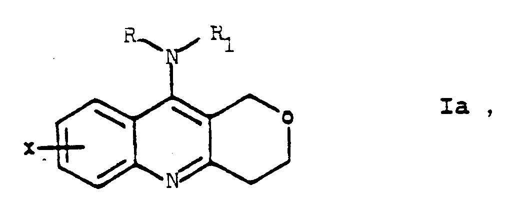 worin R und R₁ wie oben definiert sind, oder
c) Umsetzen einer Verbindung der Formel VI 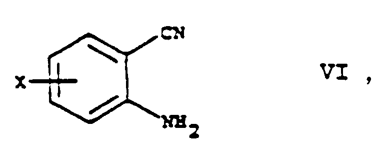 worin X wie oben definiert ist, mit Tetrahydro-4H-pyran-4-on zur Ausbildung einer Verbindung der Formel Ib

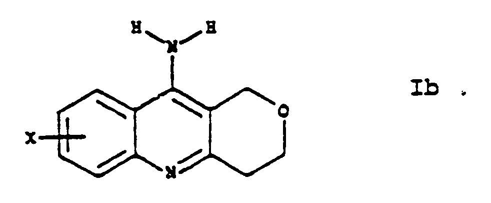

2. Verfahren nach Anspruch 1, worin A für CH₂ steht, Y die Bedeutung O oder S hat, R Wasserstoff ist, R₁ für Wasserstoff oder Phenyl-C₁-C₆-alkyl steht, worin das Phenyl wie in Anspruch 1 definiert substituiert sein kann, R₂ Wasserstoff bedeutet und n den Wert 2 hat.

3. Verfahren nach Anspruch 2, worin X Wasserstoff, Niederalkyl oder Trifluormethyl bedeutet.

4. Verfahren nach Anspruch 1, worin 9-Amino-2,3-dihydrothieno[3,2-b]chinolin hergestellt wird.

5. Verfahren nach Anspruch 1, worin 9-Amino-1,3-dihydrothieno[3,4-b]chinolin hergestellt wird.

6. Verfahren nach Anspruch 1, worin 10-Amino-3,4-dihydro-1H-pyrano[4,3-b]chinolin hergestellt wird.

7. Verfahren nach Anspruch 1, worin 10-Amino-3,4-dihydro-1H-thiopyrano[4,3-b]chinolin hergestellt wird.

8. Verfahren zur Herstellung einer Verbindung mit der Formel II 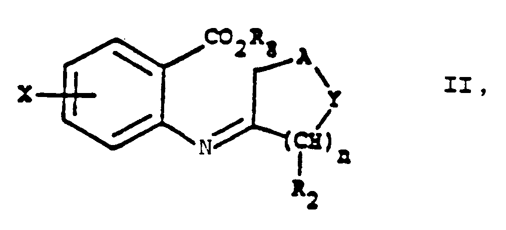 worin n, A, X und Y wie in Anspruch 1 definiert sind, R₈ Wasserstoff oder C₁-C₆-Alkyl bedeutet und R₂, R₃ und R₇ unabhängig voneinander Wasserstoff oder C₁-C₆-Alkyl darstellen, oder zwei dieser Reste gemeinsam eine Methylen- oder Ethylengruppe ausbilden, die einen Teil eines Ringes mit wenigstens fünf Atomen darstellt, welches ein Umsetzen einer Verbindung der Formel IV 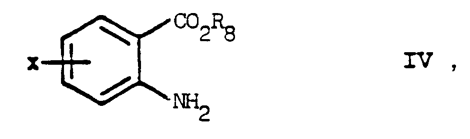 worin X und R₈ wie oben definiert sind, mit einer Verbindung der Formel VII 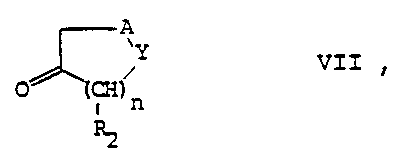 worin A, Y, R₂ und n wie oben definiert sind, umfaßt.

9. Verfahren zur Herstellung einer Verbindung der Formel III 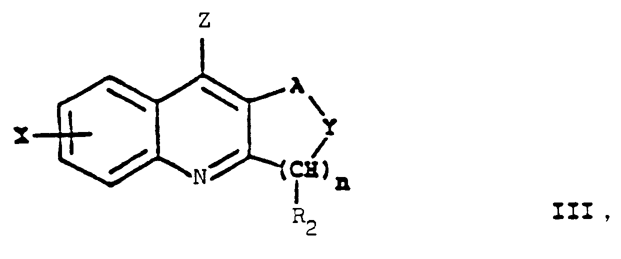 worin n, A, X und Y wie in Anspruch 1 definiert sind, Z für Halogen, Hydroxy oder C₁-C₆-Alkoxy steht; und R₂, R₃ und R₇ unabhängig voneinander Wasserstoff oder C₁-C₆-Alkyl darstellen, oder zwei dieser Reste gemeinsam eine Methylen- oder Ethylengruppe ausbilden, die einen Teil eines Ringes aus wenigstens fünf Atomen darstellen, mit der Maßgabe, daß dann, wenn A für CH₂ steht, Y für NCH₃ steht, (CHR₂)ₙ für CH₂CH₂ steht und X Wasserstoff, CH₃, Cl, Br oder NO₂ darstellt, Z nicht für Cl steht, welches Verfahren umfaßt:
a) Cyclisieren einer Verbindung der Formel II 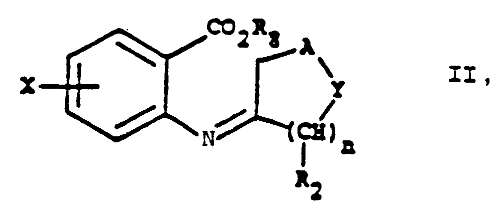 worin A, Y, X, R₂, R₈ und n wie oben definiert sind, in Gegenwart von Phosphorpentoxid zur Ausbildung einer Verbindung der Formel III, worin Z Hydroxy bedeutet, und
b) gegebenenfalls Umsetzen einer Verbindung der Formel III, worin Z Hydroxy bedeutet, mit einem Gemisch aus POCl₃ und PCl₅ oder POBr₃ und PBr₅ zur Ausbildung einer Verbindung der Formel III, worin Z für Cl bzw. Br steht, und
c) gegebenenfalls Überführen einer Verbindung der Formel III, worin Z Chlor darstellt, nach in der Technik bekannten Standardverfahren in eine Verbindung der Formel III, worin Z Fluor oder Iod bedeutet.

10. Verwendung einer Verbindung nach Anspruch 1 zur Herstellung eines Medikaments mit der Wirksamkeit der Erhöhung der cholinergen Funktionen in Säugetieren.

## Revendications (Revendications pour l'(les) Etat(s) contractant(s) suivant(s): AT, BE, CH, DE, FR, GB, IT, LI, LU, NL, SE)

1. Composé de formule I 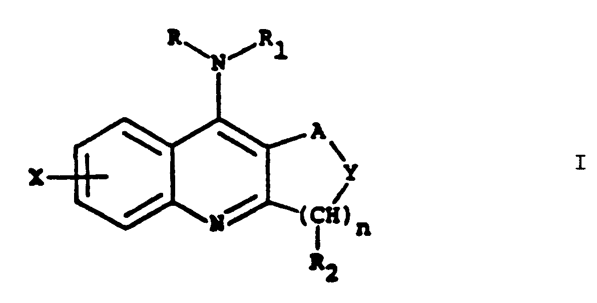 dans laquelle n vaut de 1 à 4; R est un atome d'hydrogène ou un groupe alkyle en C₁-C₆ ou alkyl(C₁-C₆)-carbonyle; R₁ est un atome d'hydrogène ou un groupe alkyle en C₁-C₆, alkyl(C₁-C₆)-carbonyle, dialkyl(C₁-C₆)amino-alkyle(C₁-C₆) ou phényle, phényl-alkyle(C₁-C₆), diphényl-alkyle(C₁-C₆), phényl-alkyle(C₁-C₆) ponté par un atome d'oxygène ou diphényl-alkyle(C₁-C₆) ponté par un atome d'oxygène, le fragment phényle pouvant être substitué par 1, 2 ou 3 substituants qui sont chacun, indépendamment, un atome d'halogène ou un groupe alkyle en C₁-C₆, alcoxy en C₁-C₆, hydroxy, trifluorométhyle, phénoxy ou benzyloxy; A est une liaison directe ou un groupe (CHR₃)ₘ, m valant de 1 à 3 ; X est un atome d'hydrogène ou d'halogène ou un groupe alkyle en C₁-C₆, cycloalkyle en C₃-C₇, alcoxy en C₁-C₆, hydroxy, nitro, trifluorométhyle, formyle, alkyl(C₁-C₆)-carbonyle, un groupe phénylcarbonyle dans lequel le fragment phényle peut être substitué par 1, 2 ou 3 substituants qui sont chacun, indépendamment, un atome d'halogène ou un groupe alkyle en C₁-C₆, alcoxy en C₁-C₆, hydroxy, trifluorométhyle, phénoxy ou benzyloxy; un groupe -SH, alkyl(C₁-C₆)-thio, -NHCOR₄ ou -NR₅R₆, R₄ étant un atome d'hydrogène ou un groupe alkyle en C₁-C₆, et R₅ et R₆ étant indépendamment un atome d'hydrogène ou un groupe alkyle en C₁-C₆ ou cycloalkyle en C₃-C₇; Y est O, S ou NR₇; et chaque groupe R₂, chaque groupe R₃ et chaque groupe R₇ sont indépendamment un atome d'hydrogène ou un groupe alkyle en C₁-C₆, ou, pris deux à la fois, forment un groupe méthylène ou éthylène faisant partie d'un cycle comportant au moins 5 atomes; étant entendu que lorsque A est CH₂, Y est NCH₃, (CHR₂)ₙ est CH₂CH₂, X est H, CH₃, Cl, Br ou NO₂ et R est H, R₁ n'est pas H ni le groupe méthyle, éthyle, propyle, butyle ou benzyle, que lorsque A est -CH₂ ou -CHR'-, Y est NH ou NR' et (CHR₂)ₙ est -CHCH₂- ou -CH₂CHR', le groupe -NRR₁ n'est pas -NH₂, -NHC₆H₅ ni un groupe dialkyl(C₁-C₆)aminoalkyl(C₁-C₆)amino, chaque radical R' étant indépendamment un groupe alkyle en C₁-C₆, que lorsque A est CH₂, Y est NH ou NR' et (CHR₂)ₙ est -(CH₂)₃- ou -CHR'CH₂CH₂-, le groupe -NRR₁ n'est pas -NH₂ et que lorsque A est -CH₂CH₂-, Y est NH ou NR' et (CHR₂)ₙ est -CH₂CH₂- ou -CHR'CH₂-, le groupe -NRR₁ n'est pas -NH₂; stéréoisomères, isomères optiques et géométriques de celui-ci, ou sel d'addition avec un acide pharmaceutiquement acceptable de celui-ci.

2. Composé selon la revendication 1, dans lequel A est CH₂, Y est O ou S, R est un atome d'hydrogène, R₁ est un atome d'hydrogène ou un radical phényl-alkyle(C₁-C₆) dans lequel le fragment phényle peut être substitué comme défini dans la revendication 1, R₂ est un atome d'hydrogène et n est 2.

3. Composé selon la revendication 2, dans lequel X est un atome d'hydrogène ou un groupe alkyle en C₁-C₆ ou trifluorométhyle.

4. Composé selon la revendication 1, qui est la 9-amino-2,3-dihydrothiéno[3,2-b[quinoléine.

5. Composé selon la revendication 1, qui est la 9-amino-1,3-dihydrothiéno[3,4-b]quinoléine.

6. Composé selon la revendication 1, qui est la 10-amino-3,4-dihydro-1H-pyranno[4,3-b]quinoléine.

7. Composé selon la revendication 1, qui est la 10-amino-3,4-dihydro-1H-thiopyranno[4,3-b]quinoléine.

8. Procédé pour la préparation d'un composé selon la revendication 1, comprenant
a) la mise en réaction d'un composé de formule III 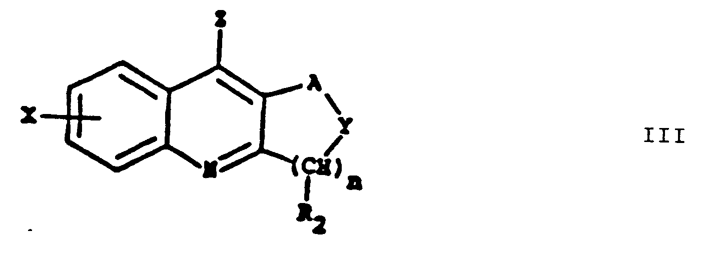 dans laquelle A, Y, R₂, et n sont tels que définis et Z est un atome d'halogène, avec une amine de formule V
HNRR₁ V
dans laquelle R et R₁ sont tels que définis, pour l'obtention d'un composé de formule I, ou
b) le chauffage d'un composé de formule IV 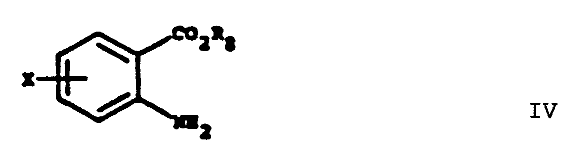 dans laquelle R₈ est un atome d'hydrogène ou un groupe alkyle inférieur, avec un mélange de pentoxyde de phosphore, de N,N-diméthylcyclohexylamine et du chlorhydrate d'une amine de formule HNRR₁, dans laquelle R et R₁ sont tels que définis, et l'addition de tétrahydro-4H-pyranno-4-one, pour l'obtention d'un composé de formule Ia, 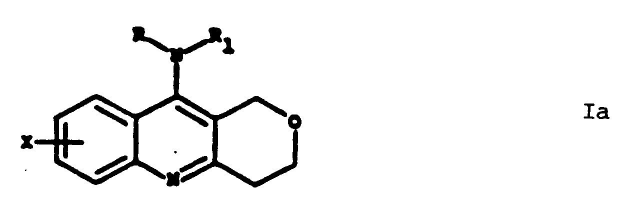 dans laquelle R et R₁ sont tels que définis, ou
c) la mise en réaction d'un composé de formule VI 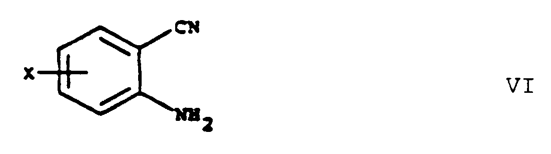 dans laquelle X est tel que défini, avec de la tétrahydro-4H-pyranno-4-one, pour l'obtention d'un composé de formule Ib

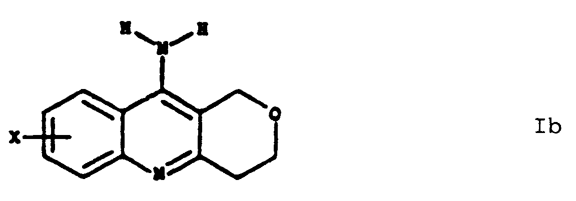

9. Composition pharmaceutique comprenant, en tant que composants actifs, un composé tel que défini dans la revendication 1, et un véhicule pharmaceutiquement acceptable.

10. Utilisation d'un composé tel que défini dans la revendication 1, pour la fabrication d'un médicament ayant l'activité d'augmentation de la fonction cholinergique chez les mammifères.

11. Composé de formule II 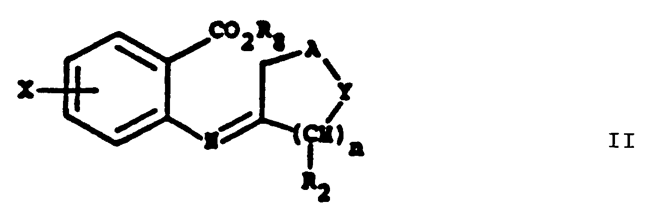 dans laquelle n, A, X et Y sont tels que définis dans la revendication 1, R₈ est un atome d'hydrogène ou un groupe alkyle en C₁-C₆; et R₂, R₃ et R₇ sont indépendamment un atome d'hydrogène ou un groupe alkyle en C₁-C₆, ou, pris deux à la fois, forment un groupe méthylène ou éthylène faisant partie d'un cycle ayant au moins 5 atomes.

12. Composé de formule III 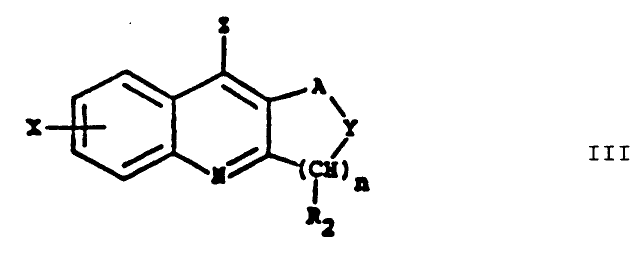 dans laquelle n, A, X et Y sont tels que définis dans la revendication 1, Z est un atome d'halogène ou un groupe hydroxy ou alcoxy en C₁-C₆; et R₂, R₃ et R₇ sont indépendamment un atome d'hydrogène ou un groupe alkyle en C₁-C₆, ou, pris deux à la fois forment un groupe méthylène ou éthylène faisant partie d'un cycle ayant au moins 5 atomes, étant entendu que lorsque A est CH₂, Y est NCH₃, (CHR₂)ₙ est CH₂CH₂ et X est H, CH₃, Cl, Br ou NO₂, Z n'est pas Cl.

## Revendications (Revendications pour l'(les) Etat(s) contractant(s) suivant(s): ES, GR)

1. Procédé pour la préparation d'un composé de formule I 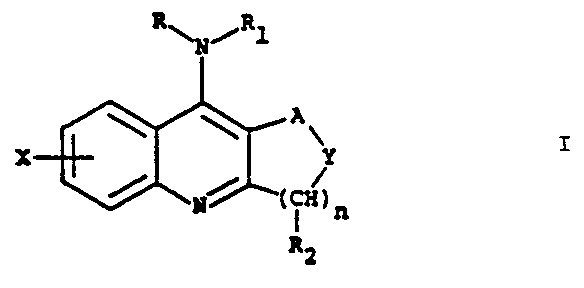 dans laquelle n vaut de 1 à 4; R est un atome d'hydrogène ou un groupe alkyle en C₁-C₆ ou alkyl(C₁-C₆)-carbonyle; R₁ est un atome d'hydrogène ou un groupe alkyle en C₁-C₆, alkyl(C₁-C₆)-carbonyle, dialkyl(C₁-C₆)amino-alkyle(C₁-C₆) ou phényle, phényl-alkyle(C₁-C₆), diphényl-alkyle(C₁-C₆), phényl-alkyle(C₁-C₆) ponté par un atome d'oxygène ou diphényl-alkyle(C₁-C₆) ponté par un atome d'oxygène, le fragment phényle pouvant être substitué par 1, 2 ou 3 substituants qui sont chacun, indépendamment, un atome d'halogène ou un groupe alkyle en C₁-C₆, alcoxy en C₁-C₆, hydroxy, trifluorométhyle, phénoxy ou benzyloxy; A est une liaison directe ou un groupe (CHR₃)ₘ, m valant de 1 à 3 ; X est un atome d'hydrogène ou d'halogène ou un groupe alkyle en C₁-C₆, cycloalkyle en C₃-C₇, alcoxy en C₁-C₆, hydroxy, nitro, trifluorométhyle, formyle, alkyl(C₁-C₆)-carbonyle, un groupe phénylcarbonyle dans lequel le fragment phényle peut être substitué comme indiqué plus haut, un groupe -SH, alkyl(C₁-C₆)-thio, -NHCOR₄ ou -NR₅R₆, R₄ étant un atome d'hydrogène ou un groupe alkyle en C₁-C₆, et R₅ et R₆ étant indépendamment un atome d'hydrogène ou un groupe alkyle en C₁-C₆ ou cycloalkyle en C₃-C₇; Y est O, S ou NR₇; et chaque groupe R₂, chaque groupe R₃ et chaque groupe R₇ sont indépendamment un atome d'hydrogène ou un groupe alkyle en C₁-C₆, ou, pris deux à la fois, forment un groupe méthylène ou éthylène faisant partie d'un cycle comportant au moins 5 atomes; étant entendu que lorsque A est CH₂, Y est NCH₃, (CHR₂)ₙ est CH₂CH₂, X est H, CH₃, Cl, Br ou NO₂ et R est H, R₁ n'est pas H ni le groupe méthyle, éthyle, propyle, butyle ou benzyle, que lorsque A est -CH₂ ou -CHR'-, Y est NH ou NR' et (CHR₂)ₙ est -CH₂CH₂- ou -CH₂CHR', le groupe -NRR₁ n'est pas -NH₂, -NHC₆H₅ ni un groupe dialkyl(C₁-C₆)amino-alkyl(C₁-C₆)amino, chaque radical R' étant indépendamment un groupe alkyle en C₁-C₆, que lorsque A est CH₂, Y est NH ou NR' et (CHR₂)ₙ est -(CH₂)₃- ou -CHR'CH₂CH₂-, le groupe -NRR₁ n'est pas -NH₂ et que lorsque A est -CH₂CH₂-, Y est NH ou NR' et (CHR₂)ₙ est -CH₂CH₂- ou -CHR'CH₂-, le groupe -NRR₁ n'est pas -NH₂; de ses stéréoisomères, isomères optiques et géométriques, ou d'un sel d'addition avec un acide pharmaceutiquement acceptable de celui-ci, comprenant
a) la mise en réaction d'un composé de formule III 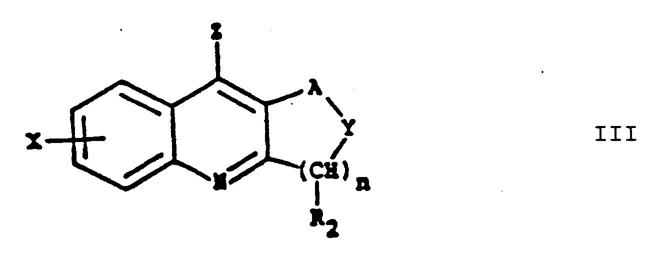 dans laquelle A, Y, R₂ et n sont tels que définis et Z est un atome d'halogène, avec une amine de formule V
HNRR₁ V
dans laquelle R et R₁ sont tels que définis, pour l'obtention d'un composé de formule I, ou
b) le chauffage d'un composé de formule IV 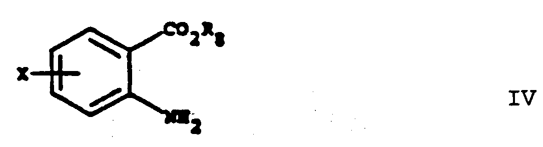 dans laquelle R₈ est un atome d'hydrogène ou un groupe alkyle inférieur, avec un mélange de pentoxyde de phosphore, de N,N-diméthylcyclohexylamine et du chlorhydrate d'une amine de formule HNRR₁, dans laquelle R et R₁ sont tels que définis, et l'addition de tétrahydro-4H-pyranno-4-one, pour l'obtention d'un composé de formule Ia, 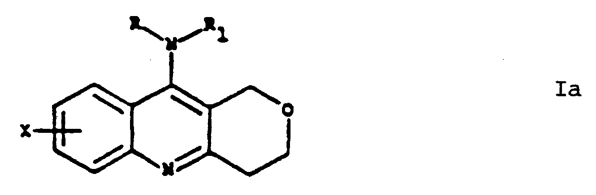 dans laquelle R et R₁ sont tels que définis, ou
c) la mise en réaction d'un composé de formule VI 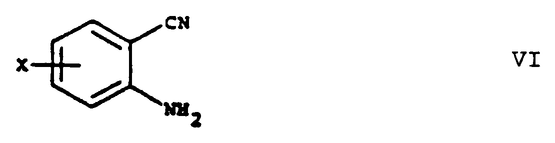 dans laquelle X est tel que défini, avec de la tétrahydro-4H-pyranno-4-one, pour l'obtention d'un composé de formule Ib

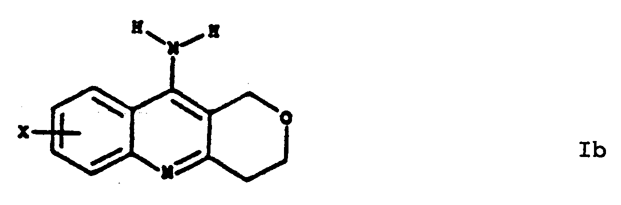

2. Composé tel que défini dans la revendication 1, dans lequel A est CH₂, Y est O ou S, R est un atome d'hydrogène, R₁ est un atome d'hydrogène ou un radical phényl-alkyle(C₁-C₆) dans lequel le fragment phényle peut être substitué comme défini dans la revendication 1, R₂ est un atome d'hydrogène et n est 2.

3. Procédé selon la revendication 2, dans lequel X est un atome d'hydrogène ou un groupe alkyle inférieur ou trifluorométhyle.

4. Procédé selon la revendication 1, dans lequel on prépare la 9-amino-2,3-dihydrothiéno[3,2-b]quinoléine.

5. Procédé selon la revendication 1, dans lequel on prépare la 9-amino-1,3-dihydrothiéno[3,4-b]quinoléine.

6. Procédé selon la revendication 1, dans lequel on prépare la 10-amino-3,4-dihydro-1H-pyranno[4,3-b]quinoléine.

7. Procédé selon la revendication 1, dans lequel on prépare la 10-amino-3,4-dihydro-1H-thiopyranno[4,3-b]quinoléine.

8. Procédé pour la préparation d'un composé de formule II 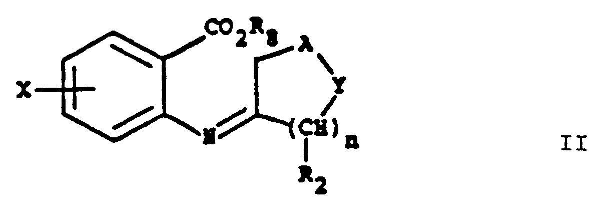 dans laquelle n, A, X et Y sont tels que définis dans la revendication 1, R₈ est un atome d'hydrogène ou un groupe alkyle en C₁-C₆; et R₂, R₃ et R₇ sont indépendamment un atome d'hydrogène ou un groupe alkyle en C₁-C₆, ou, pris deux à la fois, forment un groupe méthylène ou éthylène faisant partie d'un cycle ayant au moins 5 atomes,
comprenant la mise en réaction d'un composé de formule IV 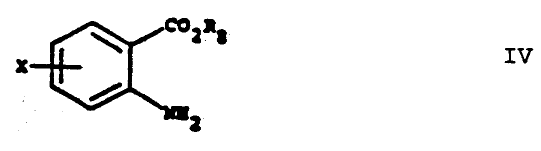 dans laquelle X et R₈ sont tels que définis plus haut, avec un composé de formule VII 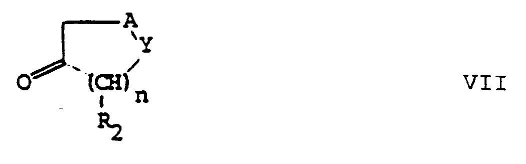 dans laquelle A, Y, R₂ et n sont tels que définis.

9. Procédé pour la préparation d'un composé de formule III 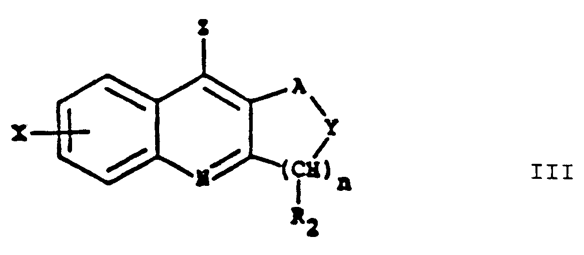 dans laquelle n, A, X et Y sont tels que définis dans la revendication 1, Z est un atome d'halogène ou un groupe hydroxy ou alcoxy en C₁-C₆; et R₂, R₃ et R₇ sont indépendamment un atome d'hydrogène ou un groupe alkyle en C₁-C₆, ou, pris deux à la fois forment un groupe méthylène ou éthylène faisant partie d'un cycle ayant au moins 5 atomes, étant entendu que lorsque A est CH₂, Y est NCH₃, (CHR₂)ₙ est CH₂CH₂ et X est H, CH₃, Cl, Br ou NO₂, Z n'est pas Cl, comprenant
a) la cyclisation d'un composé de formule II 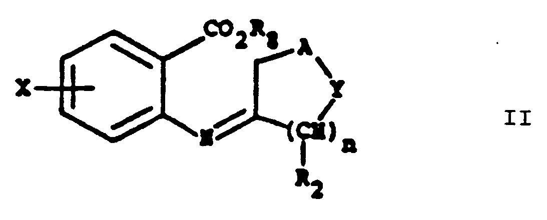 dans laquelle A, Y, X, R₂, R₈ et n sont tels que définis, en présence de pentoxyde de phosphore, pour l'obtention d'un composé de formule III dans laquelle Z est un groupe hydroxy, et
b) éventuellement la mise en réaction d'un composé de formule III, dans lequel Z est le groupe hydroxy, avec un mélange de POCl₃ et PCl₅ ou de POBr₃ et PBr₅, pour l'obtention d'un composé de formule III dans lequel Z est Cl ou Br, respectivement, et
c) éventuellement la conversion d'un composé de formule III dans lequel Z est un atome de chlore, selon des méthodes classiques connues dans la technique, pour l'obtention d'un composé de formule III dans lequel Z est un atome de fluor ou d'iode.

10. Utilisation d'un composé tel que défini dans la revendication 1, pour la fabrication d'un médicament ayant l'activité d'augmentation de la fonction cholinergique chez les mammifères.
